# EUROPEAN PATENT SPECIFICATION

(11) **EP 3 773 352 B1**
(45) Date of publication and mention of the grant of the patent: **23.07.2025**
(21) Application number: 19717597.9
(22) Date of filing: 09.04.2019
(51) Int. Cl.: A61F 2/50

(54) **PROSTHESIS CASTING SYSTEM**
PROTHESEN ABFORMSYSTEM
SYSTEME DE MOULAGE POUR UNE PROTHESE

(30) Priority: 09.04.2018 GB 201805869
(43) Date of publication of application: 17.02.2021
(73) Proprietor: University of Strathclyde, Glasgow G1 1XQ (GB)
(72) Inventor: MCGARRY, Anthony, Glasgow G1 1QE (GB); ASLANI, Navid, Glasgow G1 1QE (GB)
(74) Representative: Marks & Clerk LLP
(86) International application number: PCT/GB2019/051025
(87) International publication number: WO 2019/197814

(56) References cited:
- DE-C1- 4 328 380
- US-A- 5 432 703
- US-A1- 2014 276 094
- US-A1- 2018 036 144

## Description

### Field of the invention

The present disclosure relates to an apparatus for determining, e.g., scanning, the shape of a body part, such as a limb or residual limb, in particular, but not exclusively, while pressure is applied to the body part, and to the design and manufacture of prostheses designed to fit the scanned body part.

### Background to the invention

A prosthetic socket forms the mechanical coupling between a human residual limb and an artificial limb. Socket fit is arguably the most important design parameter in the manufacture of prostheses for persons with upper and lower-limb amputation. The socket transfers load under static and dynamic conditions to facilitate comfortable walking and activities of daily life. A loose, tight or incorrectly shaped coupling between the socket and the residual limb may cause laxity or excessive interface forces accordingly. Both laxity and excessive forces increase shear stress and may result in pain, discomfort, soft tissue breakdown and a reluctance to use of the inefficient prosthesis.

Typically, the shape of the residual limb is captured using plaster of Paris. This is conducted in an artisan way and relies on the hand skills and judgement of an experienced clinician. As the plaster cast is taken in an unloaded position, the soft tissue of the residual limb is not in the same shape as it would be when a prosthetic is fitted and in use. Further modification of the shape captured with the plaster cast is required to ensure the correct fit when loaded.

3D scanners can also be used to capture the shape of a residual limb. However, current methods suffer from a number of disadvantages such as costs and scanning in unloaded conditions. For example, when a shape of a residual limb is captured in unloaded conditions, some adjustments and/or rectifications are needed after capturing the shape. These corrections which are carried out by operators represent a possible source of error.

DE4328380C1 (LAUTH) describes a method for the fabrication and/or dimensional adjustment of aids, in particular orthopaedic aids, designed to fit parts of the living body by means of a fitting surface. The part of the body to which the aid is to be fitted is first immobilized. The immobilized body part is then covered with a flexible membrane which forms part of a fixed pressure-medium chamber. The pressure-medium chamber is brought to the fitting pressure with the pressure medium and pressed with the fitting pressure against the part of the body to which the aid is to be fitted, compressing the body part. By means of the membrane pressed on to it, the surface of the body part concerned is plotted using a fixed coordinate system. The aid is fabricated in accordance with the fitting surface thus determined. Also described are devices for carrying out the method.

US2018036144A1 (RADSPIELER) describes a medical apparatus for use in manufacturing a plaster impression of limb stump, in particular of a lower stump, wherein the apparatus comprises a pressure vessel with a fluid chamber of pressure chamber for receiving or storing a fluid being under pressure, wherein the pressure vessel is limited against an exterior, wherein the pressure vessel comprises an insertion opening for the insertion of a limb stump into the interior of the pressure vessel and a fluid-tight membrane made of two materials, which are arranged to form or limit the fluid chamber or the pressure chamber. In addition, it describes a system having a medical apparatus and at least one contact surface. Furthermore, describing a method. This document discloses the features of the preamble of claim 1.

### Summary

According to a first aspect of the present disclosure there is provided a scanning apparatus according to claim 1.

In use, the container may be arranged to receive a pressurised fluid. The pressurised fluid may be either a pressurised liquid or a pressurised gas. It will be understood that, in use, the container will be pressured using either a liquid or a gas. However, the container may advantageously be capable of receiving a pressurised liquid. By such provision, in circumstances where it may be advantageous to use a pressurised liquid, rather than a pressurised gas, the apparatus may be capable of scanning the body part under pressure using a pressurised liquid.

The container may be arranged to receive both pressurised liquid and a pressurised gas. Typically, in use, the container may receive one of liquid and gas, but may be capable of receiving either liquid or gas, depending on the specific parameters required at the time of the scan. The container may be configured to hold a pressurised liquid, and optionally may be configured to hold a pressurized gas.

Advantageously, the use of a pressurised liquid may be safer than use of a pressurised gas. For example, while liquids such as water are incompressible, gases such as air are compressible. Thus, under use of compressible gases, failure or disconnection of one or more parts in the apparatus while under pressure could pose a risk of damage to surrounding equipment and/or of injury to standers-by. The use of a pressurised liquid, e.g. water, may reduce risks of damage and/or may improve safety.

Advantageously, the use of an uncompressible liquid may also allow the container to be pressurised more quickly. An uncompressible liquid will pressurise almost instantly on connection to a pump, while a gas has to compress before it can reach the desired pressure. The pressure in an uncompressible liquid may also be more stable. For example, if the body part is moved, the uncompressible liquid may move around the body part, whereas a compressible gas may be associated with less stable pressure. For example, a gas may be compressed on one side of the body part and may expand on the other side of the body part before the pressure equilibrates.

A container capable of holding a pressurised liquid may also be easier to manufacture than a container capable of holding a pressurised gas. Water molecules are larger than oxygen or nitrogen molecules, and water is more viscous than air, so a water-tight container may be easier to manufacture and/or may be more reliable or easier to operate than an air-tight container.

The liquid may comprise an aqueous liquid. Conveniently, the liquid may comprise or may be water.

The gas may comprise or may be air.

Also disclosed herein is a scanning apparatus for scanning a body part, the scanning apparatus comprising:
a container configured to receive the body part and to receive a flow of fluid, wherein the container comprises a longitudinal axis along which the cross-section of the container changes.

In use, the flow of fluid and/or the container may be configured to apply pressure to or on at least a portion of an object located in the container, for example, a portion of the body part.

The container may be configured to receive the body part substantially along the longitudinal axis of the container. In use, the change in cross-section of the container along the longitudinal axis may advantageously cause a change in the pressure of the fluid along the body part. In use, the pressure applied by the fluid to the body part may vary and/or may depend on the cross-section of the container, e.g. on a dimension, shape, area and/or width thereof. Control and/or design of the cross-section of the container may result in control of the pressure of the fluid within the container and/or on or along the body part.

The container may be configured so as to provide a pressure gradient along to limb, when a/the fluid, e.g. pressurised fluid, is fed or pumped into the container.

The change in the cross-section of the container along the longitudinal axis may comprise changes in the shape of the cross-section and/or changes in the area of the cross-section. At least part of the container may have a circular, oval, square or rectangular cross-section along the longitudinal axis.

Typically, the shape of a cross-section of the container may be substantially constant or unchanged along at least part of its longitudinal axis or length, e.g. along its entire longitudinal axis or length. A dimension or size, e.g. area and/or circumference of a cross-section of the container may vary along at least part of its longitudinal axis or length, e.g. along its entire longitudinal axis or length.

The container may comprise a first end and a second end. The container may comprise a first section at, near or containing the first end. The container may comprise a second section at, near or containing the second end. The container may comprise an intermediate section. The intermediate section may be located between the first end and the second end and/or between the first section and the second section. The intermediate section may comprise or may define a middle or central section, which may be substantially half way between the first end and the second end.

The first end and the second end may have an identical or substantially identical cross-section along the longitudinal axis, or they may have different cross-sections along the longitudinal axis. The first section end and the second section may have an identical or substantially identical cross-section along the longitudinal axis, or they may have different cross-sections along the longitudinal axis. The intermediate section may have a different cross-section along the longitudinal axis compared to the first end or the second end.

A dimension or size, e.g. area, diameter or circumference, of a cross-section in the intermediate section may be different, e.g. smaller, than a dimension or size, e.g. area, width or circumference, of a cross-section in the first section. A dimension or size, e.g. area, diameter or circumference, of a cross-section in the intermediate section may be different, e.g. smaller, than a dimension or size, e.g. area, width or circumference, of a cross-section in the second section.

The container may comprise walls. The walls may comprise at least partially concave walls or at least partially convex walls. The container may have adjustable walls. The container and/or the container walls may be configurable to control the cross-section of the container along the longitudinal axis. The container may comprise or may define a venturi container.

The cross-section of the container along the longitudinal axis may have or may define a width. The width of any cross-section along the longitudinal axis may be defined as the distance, in a direction perpendicular to the longitudinal axis, between opposing walls of the container. The cross-section of the container along the longitudinal axis may have or may define a first width and a second width. The first width may be defined as the largest distance, in a first direction in a plane perpendicular to the longitudinal axis, between opposing walls of the container. The second width may be defined as the shortest distance, in a second direction in a plane perpendicular to the longitudinal axis, between opposing walls of the container. When the container is circular in cross-section, the first width and the second width may be substantially identical.

Any of the first end or first section, the intermediate section or the second end or second section may have the largest cross-section along the longitudinal axis. Any of the first end or first section, the intermediate section or the second end or second section may have the smallest cross-section along the longitudinal axis. Any of the first end or first section, the intermediate section or the second end or second section may have the largest first width and/or second width. Any of the first end or first section, the intermediate section or the second end or second section may have the narrowest first width and/or second width.

The intermediate section may typically comprise a waist section and/or may comprise the smallest cross-section of the container along the longitudinal axis.

The change in the cross-section of the container along the longitudinal axis may comprise a step change. The cross-section of the container along the longitudinal axis may comprise one or more changes along the longitudinal axis.

The change in the cross-section of the container along the longitudinal axis may comprise a gradual change or a progressive change. The cross-section of the container along the longitudinal axis may change gradually from the first end to the second end. For example, the cross-section may be gradually reduced from a/the first end to a/the middle or intermediate section of the container, and the cross-section may be gradually reduced gradually from a/the second end to a/the middle or intermediate section of the container.

The change in the cross-section of the container along the longitudinal axis may comprise a stepped change. The cross-section of the container along the longitudinal axis may change in a stepped manner from the first end to the second end. For example, the cross-section may be reduced from a/the first end to a/the middle or intermediate section of the container via one or more steps, and the cross-section may be reduced gradually from a/the second end to a/the middle or intermediate section of the container via one or more steps. The cross-section between two consecutive steps may be constant, e.g., the steps provide the change in cross-section. The cross-section between two consecutive steps may be variable, e.g., the steps provide a change in cross-section, and the cross-section of the container along the longitudinal axis between at least two consecutive steps may comprise a gradual change or a progressive change.

The container may be rotationally symmetric along the longitudinal axis. The container may be rotationally asymmetric along the longitudinal axis.

The container may be configured to receive a flow of fluid from the first end, through the intermediate section, and out the second end. The first end may define an inlet. The second end may define an outlet.

The fluid may comprise a liquid or a gas. The liquid may comprise an aqueous liquid, e.g. water. The gas may comprise an inert gas or mixture of gases. The gas may comprise air.

The height of the container, or the distance from the first end to the second end, may be in the range of about 0.2m to about 1m, e.g. between approximately 0.3m and 0.5m. A cross-section of the container may be in the range of about 0.2 - 1 m, e.g. about 0.3 - 0.5 m.

The scanning apparatus comprises a collapsible container configurable to receive the body part and to receive the first fluid.

The collapsible container may advantageously be lightweight and/or portable. This may be particularly beneficial for use in developing countries, and/or in rural areas or places which are remote from medical facilities such as hospitals. A lightweight and/or portable collapsible container may allow easier access to prosthetics for people in such rural or remote locations. Such a container may allow "point of care" treatment of patients who do not wish to travel to a medical centre or hospital, and/or where capacity and/or resources in such medical centre or hospital are limited.

The collapsible container may advantageously be configurable to receive body parts of different shapes and sizes, e.g. legs, arms, hips, etc.

The collapsible container may be configurable between at least two configurations. The collapsible container may be configurable between a collapsed configuration and an extended/deployed configuration. The collapsed configuration may comprise a stowed configuration, e.g. when not in use. The extended configuration may comprise a deployed and/or active configuration e.g. when in use or when ready for use. It will be understood that the collapsible container may be in use or ready for use in a partially extended configuration. For example, the collapsible container may be configured to receive the body part and a first fluid when partially extended/deployed. The collapsible container may at least partially define a first volume. The collapsible container may be configurable to receive the body part and a first fluid in the first volume. The collapsible container may be deployable or extendable by the pressurisation of the first fluid.

The collapsible container may be concertinaed, for example the wall(s) of the collapsible container may be concertinaed. The collapsible container may be expandable or collapsible with a concertina action.

The collapsible container isat least partially flexible. A flexible collapsible container may advantageously fit around different body parts easily.

The scanning apparatus may comprise a frame configured to support the collapsible container. The frame may be deployable between a first or stowed configuration and a second or deployed configuration. The collapsible container may be attachable to the frame. In a deployed configuration, the frame may provide support for the collapsible container and/or may define the deployed and/or active configuration.

The collapsible container may be configurable and/or may be provided, in use, around the frame. The frame may be configurable and/or may be provided, in use, inside the collapsible container. The collapsible container and/or the frame may be free-standing.

The scanning apparatus may comprise a plate. The plate may be substantially planar. The frame may be mounted or attached to a first side of the plate. The collapsible container may be mounted or attached to the first side of the plate. The collapsible container may be mounted or attached to the plate around the frame. In a/the extended/deployed configuration, the collapsible container may extend around the frame. The first volume may be at least partially defined by the plate and the collapsible container. When the collapsible container is in the extended/deployed configuration, the frame may be within the first volume. The plate may separate the first volume from the remainder of the scanning apparatus.

The collapsible container may comprise one or more walls.

The collapsible container may comprise an inner wall and an outer wall.

The inner wall may be tubular, e.g. in a deployed configuration. The inner wall may define or comprise an inner volume. The inner volume may be configurable to receive the body part and/or the first fluid.

The outer wall may be tubular, e.g. in a deployed configuration. The inner wall may be within or may be provided inwards of the outer wall. The outer wall may surround the inner wall. The inner wall and outer wall may be separated by an outer volume. The outer volume may be between the inner wall and the outer wall. The outer volume may be annular, for example the outer volume may be annular when the collapsible container is expanded or deployed, e.g. uncollapsed. The outer volume may be configured to receive a second fluid.

The collapsible container and/or outer volume may be at least partially inflatable. The collapsible container may be expandable by inflating the outer volume. The outer volume may be inflatable by feeding or pressurising a second fluid within the outer volume. The outer volume may be configured to receive a second fluid, e.g. a pressurised second fluid. The collapsible container may be collapsed by deflating the outer volume. The collapsible container and/or the outer volume may be deflatable. In use, deployment of the container from its collapsed configuration to its deployed, active, or expanded configuration may be performed by injecting or feeding a/the second fluid into the outer volume.

The first fluid and/or the second fluid may comprise a liquid or a gas. The liquid may comprise water. The gas may comprise an inert gas or mixture of gases. The gas may comprise air.

The first volume may be configurable such that body weight exerted through the body part pressurises the first fluid within the first volume.

The scanning apparatus may comprise scales. The scales may be configured to measure or determine a weight, e.g. the body weight exerted through the body part.

The apparatus, e.g. scales, may be capable of measuring distribution of weight, in use. For example, the apparatus, e.g. scales, may be capable of measuring the distribution of weight. i.e. the percentage of the subject's weight carried or taken by the side received in the container (e.g, an amputated side) and/or the percentage of the subject's weight carried or taken by the side not received in the container (.e.g., a sound side). This may improve the scientific understanding and control of loaded shape capture during the scanning procedure, as the weight distribution may be measured whilst the shape of the body part is electronically captured by the scanning apparatus under loaded conditions.

The (collapsible) container may comprise a tank. The (collapsible) container may comprise a bag.

The body part may comprise a residual limb. The body part may comprise a residual leg or residual arm. The body part may be a lower or upper limb amputation (transtibiial, transfemoral, hip, knee or ankle disarticulation, or partial foot amputation). The body part may comprise at least a portion of a/the pelvis and/or of a/the femur.

The scanning apparatus may be configured to scan the or any body part in any conventional way for scanning or determining shape and/or topography. The scanning apparatus may comprise any conventional means for assessing, determining or scanning the shape and/or topography of the body part. For example, the scanning apparatus may comprise a scanning system which may comprise a laser line scanner, a stereo vision scanner, a photogrammetry scanner and/or structured light scanner. The scanning system may comprise multiple scanners. The scanners may all be the same type of scanner, or at least some of the scanners may be different to at least some of the other scanners. Two or more scanners may be identical. Two or more scanners may be different. The scanning apparatus may be configured to scan the body part in different ways, using different scanners.

The scanning apparatus, e.g., scanning system, may comprise a light source. The scanning apparatus, e.g., scanning system, may comprise one or more light sources. The one or more light sources may comprise at least one of: a laser, a laser projector, an LED, an IR source, a white light source and/or a structured light emitter.

The scanning apparatus, e.g., scanning system, may comprise a detector. The scanning apparatus, e.g., scanning system, may comprise one or more detectors. The one or more detectors may comprise a video camera or a picture camera.

The detectors may be in fixed or known positions. For example, for use in photogrammetry, the position of each detector may be known. For use in stereo vision, the separation between two neighbouring detectors may be known. Neighbouring or adjacent detectors may have at least partially overlapping fields of view. The detectors may be moveable, such that the portion of the body part for scanning falls within the field of view of the detector. The detectors may be moveable around the container and/or longitudinally along the container.

At least some of the detectors and/or at least some of the light sources may be directed towards a longitudinal axis of the container. All of the detectors and/or all of the light sources may be directed towards a longitudinal axis of the container.

The one or more light sources and/or the one or more detectors may be configured to scan the body part.

The one or more light sources and/or the one or more detectors may be inside the (collapsible) container. The one or more light sources and/or the one or more detectors may be outside the (collapsible) container.

The scanning apparatus, e.g., scanning system, may comprise a frame. The frame may support some or all of the one or more detectors and/or some or all of the one or more light sources. The frame may be coupled to the (collapsible) container. The frame may support the (collapsible) container. The frame may be at least partially collapsible. The frame may be inside or outside the (collapsible) container. The scanning apparatus may comprise multiple frames. Each frame may be the same as the other frames.

The one or more light sources and/or the one or more detectors may be coupled to the (collapsible) container. For example, the light source and/or the detector may be attached to the container.

The (collapsible) container may be at least partially transparent. The one or more light sources may be able to project light through the (collapsible) container. The one or more detectors may be able to detect light which has passed through the (collapsible) container.

The (collapsible) container may comprise a sealing means. The sealing means may at least partially seal the (collapsible) container. The sealing means may seal around or against the body part, thereby sealing the (collapsible) container while the body part is in the (collapsible) container. The sealing means may comprise a drawstring. The sealing means may seal the (collapsible) container in an air-tight or water-tight manner.

The sealing means may comprise an air-tight and/or water-tight barrier.

The sealing means may be adjustable in size. By such provision, the sealing means may provide for body parts having different sizes, including different body parts of a subject and/or body parts of subjects being of different sizes (e.g. height, weight, age, sex, etc).

The sealing means may comprise a latex barrier. The provision of a later barrier may provide an effective seal for the container, thus allowing pressurisation, whist also providing a hygienic contact surface with the body part of the subject or patient. Advantageously, once the body part has been placed in position and loaded by the user, the latex sheath may be pressurised. Actuating means, e.g. a vacuum pump, may be provided to control and/or actuate the sealing means, e.g., latex barrier. For example, the sealing means, e.g., latex barrier may be expanded, e.g. may define a large opening into the container, prior to the body part being inserted into the apparatus. The sealing means, e.g., latex barrier may be contracted, e.g. may define a small opening into the container, once the body part has been inserted into the apparatus. By such provision, no additional shear forces are inadvertently applied to the body part to be scanned, e.g., residual limb, due to contact with the sealing means during insertion of the body part into the apparatus.

The sealing means may comprise an inflatable bladder. The inflatable bladder may be connected to and/or actuated by a pump. The pump may be a dedicated pump for the sealing means, e.g. bladder. The pump may be the same pump as the/a pump used to pressurise the contents (fluid) of the container.

When the container is configured to receive a gas, e.g. when the first fluid is a gas, the sealing means may partially seal the container. For example, when the first fluid is a gas, e.g. air, the apparatus system may not need to be perfectly sealed so long as a pump injects gas, e.g. air, into the container to provide a/the required pressure within the container. A pressure monitoring system may be used to monitor the pressure inside the container and/or adjust pressure as required, e.g. via manual or automatic adjustment to an inlet pump or to an outlet valve so as to increase or reduce pressure, respectively.

The (collapsible) container may be approximately cylindrical. The (collapsible) container may comprise a circular cross-section. The (collapsible) container may have a diameter in the range of about 0.2m to about 1m, e.g. between approximately 0.3m and 0.5m. The (collapsible) container may have a height in the range of about 0.2m to about 1m, e.g. between approximately 0.3m and 0.5m.

The scanning apparatus, e.g., scanning system, may comprise a plurality of detectors, e.g. 12 or more detectors. Increasing the number of detectors may increase the speed and/or accuracy of the scan. Each detector may point in a different direction to each other detector. Each detector may be configured to scan a different part of the body part. For example, each detector may be configured to capture an image of a different part of the body part. Each detector may be configured to scan a different part of the body part, wherein the scan of each detector partially overlaps with the scan of another detector. The detectors may be configured such that the fields of view of each detector partially overlaps with the fields of view of other detectors.

Separate scans from different detectors may be combined and/or compiled to form or to identify a/the final shape of the body part.

The scanning apparatus, e.g., scanning system, may comprise one or more moveable detector and/or one or more moveable light sources. The one or more moveable detectors and/or one or more moveable light sources may be moveable relative to the scanning apparatus and/or relative to the (collapsible) container. The one or more moveable detectors may be configured to scan different parts of the body part by moving around the body part, for example, by moving relative to the body part. The one or more moveable light sources may be configured to illuminate different parts of the body part by moving around the body part, for example by moving relative to the body part. The one or more moveable detectors may comprise a picture camera or a video camera.

The one or more moveable detectors and/or one or more moveable light sources may be mounted, such as moveably mounted, on a guide or frame. The one or more moveable detectors and/or one or more moveable light sources may be moveable around the frame, or the frame may be moveable around the scanning apparatus and/or (collapsible) container. For example, the frame may be moveable relative to the container. The frame may be a rotating frame. The rotating frame may be configured to rotate at least partially around the scanning apparatus or the (collapsible) container.

The scanning apparatus, e.g., scanning system, may comprise a motor. The motor may be configured to move the one or more moveable detectors and/or the one or more moveable light sources relative to the scanning apparatus. The motor may be configured to move the frame relative to the scanning apparatus or (collapsible) container.

The one or more light sources and the one or more detectors may be configurable to scan the body part by laser line scanning, stereo vision, photogrammetry and/or structured light. The scan may comprise a 3D scan. The one or more light sources and the one or more detectors may be configurable to scan 360° around the body part.

The scanning apparatus may allow determination of an/the angle (e.g., in 3D) at which the body part is scanned and/or loaded, thus permitting determination of the alignment of a prosthetic element (such as foot, knee, etc). Prosthetic components typically require precise positioning (e.g., alignment) in respect to their respective prosthetic socket in order to facilitate and prolong optimum function and safety. This is typically currently facilitated using expensive bench alignment jigs which help position the socket and components relatively to each other. Commercially available miniature coupling devices consisting of pyramids and Allen keys are used to correct errors. The present arrangement may allow determination of the loaded characteristics of each portion of the limb including angle in a reliable and repeatable manner, and may allow the positioning of alignment devices correctly every time. The present system may also allow a user to 3D print an entire prosthesis, e.g., limb, repeatedly in the correct position without the need for alignment or coupling devices.

The scanning apparatus, e.g., scanning system, may comprise a pump. The pump may be configured to pressurise the contents (fluid) of the (collapsible) container. The pump may be coupled to the (collapsible) container, for example by a pipe. The pump may be configured to fill or partially fill the (collapsible) container with the fluid (liquid or gas).

The scanning apparatus, e.g., scanning system, may comprise a height adjuster. The height adjuster may comprise an adjustable mechanism. The height adjuster may be configurable to raise or lower the scanning apparatus and/or the collapsible container. This may advantageously allow users of different heights to use the apparatus, or for the apparatus to be used with residual limbs of different lengths.

The scanning apparatus, e.g., scanning system, may comprise one or more secondary detectors and/or one or more secondary light sources. The one or more secondary detectors and one or more secondary light sources may be configurable to scan a secondary body part, for example, an intact leg or intact arm. The one or more secondary detectors and one or more secondary light sources may be configured to scan the secondary body part in the same manner or using the same methods as used to scan the body part. Data obtained from the secondary detectors and/or secondary light sources may be used to help align the prosthetic leg better. It can also be used to build symmetrical cover for artificial limb. It will be appreciated that the one or more secondary detectors may comprise any of the features of the one or more detectors, and that the one or more secondary light sources may comprise any of the features of the one or more light sources.

The scanning apparatus may comprise a socket shape. The socket shape may be mountable on a support. The socket shape and/or the support may be mountable to the frame and/or the container. The socket shape and/or the support may be mountable to the frame and/or the container with an attachment, e.g. an adjustable attachment. The socket shape may be extendable. The socket shape may be movable relative to the container and/or frame when mounted to the container and/or frame.

The socket shape may comprise markers. Each marker may be different to each other marker. Each marker may be uniquely identifiable. The markers may comprise a chiral arrangement. The markers may be arranged such that the mirror image of the markers comprises a non-superimposable mirror image of the markers. The markers may be arranged such that identification of the markers uniquely identifies the position and orientation of the socket shape.

The use of a socket shape may advantageously allow the shape of portions of the body part which cannot easily be scanned to be added to the scan of the body part. By matching the shape of the socket shape to the portion of the body part which cannot be scanned, and by knowing the shape of the socket shape, determining the position and orientation of the socket shape during the scan using the markers allows the shape and orientation of the portion of the body part which cannot be scanned to be known. The portion of the body part which cannot be scanned can then be added to the 3D model of the body part.

The socket shape may be shaped to correspond to the shape of at least one of: the inner thigh; the groin; the pelvis; the ischial tuberosity; and/or the greater trochanter. The socket shape may be shaped such that the socket shape may cup the corresponding body part.

The scanning apparatus, e.g., scanning system, may comprise a computer. The computer may control the scanning apparatus, the one or more light sources, and/or the one or more detectors. The computer may automate the scanning apparatus and/or scanning system. The computer may be connected to the scanning apparatus, the one or more light sources, and/or the one or more detectors. The computer may be configured to store the scans captured by the one or more detectors. The computer may be configured to analyse the scans captured by the one or more detectors. For example, the computer may be configured to analyse images captures by the one or more detectors.

According to a second aspect of the present disclosure there is provided a method of scanning a body part according to the appended claims.

The method may comprise scanning the body part with the scanning apparatus of the first aspect of the present disclosure.

Pressurising the liquid may apply pressure to or on at least a portion of an object located in the container, for example, a portion of the body part.

The method may comprise scanning the body part with the scanning apparatus of the first aspect of the present disclosure.

The method may comprise receiving the body part in the collapsible container of the first aspect of the present disclosure. Receiving the body part in the collapsible container may comprise placing the body part in the collapsible container, and/or it may comprise extending the collapsible container around the body part.

Pressurising the fluid may apply pressure to or on at least a portion of an object located in the container, for example, a portion of the body part.

The method may comprise inflating a portion of the collapsible container. For example, the method may comprise inflating a second volume of the collapsible container to extend the collapsible container.

The method may comprise pressurising the first fluid in the collapsible container.

The method may comprise using the scanning apparatus of the first aspects of the present disclosure.

The method may comprise scanning the body part in any conventional way for scanning or determining shape and/or topography. The method may comprise assessing, determining or scanning the shape and/or topography of the body part. For example, the method may comprise laser line scanning, stereo vision scanning, photogrammetry scanning and/or structured light scanning. The method may comprise scanning the body part more than once and/or with more than one of these scanning methods. The body part may be scanned with different methods simultaneously or at different times, e.g. consecutively or sequentially.

One skilled in the art will understand that the different scanning methods may each be most suitable in different applications. For example, laser line scanning may be fast, e.g. may be the fastest method. Laser line scanning may be the most accurate method, and may have the easiest and/or fastest post-processing. Stereo vision may provide 3D shape and texture of a portion of the body part. Photogrammetry may provide good or superior texture information.

The method may comprise receiving or placing the body part in a (collapsible) container. The method may comprise sealing the (collapsible) container around or against the body part. The method may comprise sealing the (collapsible) container with the body part at least partially within the (collapsible) container. Sealing the (collapsible) container may comprise providing an air-tight seal and/or a water-tight seal, e.g. using a latex barrier and/or an inflatable bladder.

The body part may comprise a residual limb. The body part may comprise a residual leg or residual arm or any other body part.

In the second aspect, pressurising the liquid or fluid around the body part may comprise pumping the liquid or fluid in to the (collapsible) container.

Scanning the body part may comprise illuminating the body part. Illuminating the body part may comprise illuminating the body part from different angles. Illuminating the body part from different angles may comprise moving a light source relative to the body part, for example around the body part. Illuminating the body part from different angles may comprise illuminating the body part with multiple light sources. Illuminating the body part with multiple light sources may comprise illuminating different parts of the body part with multiple light sources simultaneously. Illuminating the body part may comprise illuminating the body part around 360°.

The method may comprise illuminating the body part with laser light, white light infrared radiation, ultraviolet (UV) radiation and/or structured light.

Scanning the body part may comprise detecting, determining or analysing the shape and/or topography of the body part. Scanning the body part may comprise imaging the body part. Scanning the body part may comprise scanning the illuminated body part. Scanning the body part may comprise scanning the body part from different angles. Scanning the body part from different angles may comprise moving a detector around the body part. Scanning the body part from different angles may comprise scanning the body part with multiple detectors. Scanning the body part with multiple detectors may comprise scanning different parts of the body part with multiple detectors simultaneously. Scanning the body part may comprise scanning the body part around 360°.

The method may comprise scanning the body part by imaging the body part. Imaging the body part may comprise videoing the body part and/or photographing the body part.

The method may comprise illuminating the body part and/or scanning the body part from outside the container. The method may comprise illuminating the body part and/or scanning the body part from inside the container.

Pressurising the liquid or fluid around the body part or flowing fluid along the body part may comprise pressurising the soft tissue of the body part. Pressurising the soft tissue of the body part may comprise shaping the soft tissue of the body part into a loaded condition or shape, such as the shape the body part may adopt when fitted to a prosthetic and under load. For example, shaping the soft tissue of a residual leg may comprise shaping the soft tissue of the residual leg into the shape the residual leg would adopt when a prosthetic leg is fitted to the leg and body weight is applied through the residual leg into the prosthetic.

The method may comprise using scales to determine the body weight exerted through the body part. The method may comprise adjusting the pressure of the fluid such that a predetermined or selected proportion of the user's body weight is exerted through the body part, for example from about 25% to about 400% of the user's body weight, e.g. from about 30% to about 300% of the user's body weight, e.g. from about 30% to about 200% of the user's body weight, e.g. from about 30% to about 100% of the user's body weight, e.g. from about 30% to about 80% of the user's body weight, e.g. from about 40% to about 70% of the user's body weight, e.g. about 50% of the user's body weight. The proportion of body weight exerted through the body part will change the shape of the body part, as the load applied to the body part will change. These changes in body shape may advantageously allow the body part to be scanned under different loads. Different activities may apply different loads to the body part when a prosthesis is used, for example when a user is running, walking, jumping etc. Scanning the body part whilst different proportions of body weigh are exerted through the user's body weight may advantageously allow the body part to be scanned for the design of prosthetics for different uses, e.g. for running, walking etc. One skilled in the art will understand the appropriate proportion of body weight to be exerted through the body part to achieve the desirable load applied to the body part, and therefore the desirable shape of the body part.

The method may comprise measuring distribution of weight, in use. For example, the method may comprise measuring the distribution of weight. i.e. the percentage of the subject's weight carried or taken by the side received in the container (e.g, an amputated side) and/or the percentage of the subject's weight carried or taken by the side not received in the container (.e.g., a sound side). This may improve the scientific understanding and control of loaded shape capture during the scanning procedure, as the weight distribution may be measured whilst the shape of the body part is electronically captured by the scanning apparatus under loaded conditions.

The method may comprise determining an/the angle (e.g., in 3D) at which the body part is scanned and/or loaded, thus permitting determination of the alignment of a prosthetic element (such as foot, knee, etc). Prosthetic components typically require precise positioning (e.g., alignment) in respect to their respective prosthetic socket in order to facilitate and prolong optimum function and safety. This is typically currently facilitated using expensive bench alignment jigs which help position the socket and components relatively to each other. Commercially available miniature coupling devices consisting of pyramids and Allen keys are used to correct errors. The present method may allow determination of the loaded characteristics of each portion of the limb including angle in a reliable and repeatable manner, and may allow the positioning of alignment devices correctly every time. The present method may also allow a user to 3D print an entire prosthesis, e.g., limb, repeatedly in the correct position without the need for alignment or coupling devices.

The method may comprise scanning a secondary body part. The method may comprise scanning an intact equivalent body part to the body part. For example, if the body part is a residual leg, the method may comprise scanning the other, intact, leg. Scanning the secondary body part may comprise determining the shape, size, and/or colour of the secondary body part.

The method may comprise scanning the secondary body part using any of the same methods used to scan the body part. The method may comprise receiving or placing the secondary body part in the (collapsible) container, or the method may comprise scanning the secondary body part outside the (collapsible) container. Scanning the secondary body part may comprise illuminating the body part with one or more secondary light sources and/or detecting the secondary body part with one or more secondary detectors.

The method may comprise using a socket shape. The method may comprise mounting or attaching the socket shape to the scanning apparatus, for example to the container or the frame.

The method may comprise placing or extending the socket shape into contact with the body part. The method may comprise selecting an appropriately shaped and sized socket shape for the body part. The method may comprise cupping at least a portion of the body part with the socket shape. The method may comprise scanning the body part whilst at least a portion of the body part is in contact with the socket shape.

The method may comprise scanning at least part of the socket shape during the scan. The method may comprise scanning markers on the socket shape during the scan.

The method may comprise generating a 3D model of the body part, for example generating a 3D digital model of the body part.

The method may comprise making a 3D model of the body part, such as a 3D computer model, for example a CAD model. Making the 3D model may comprise stitching together separate scans of the body parts, for example stitching together scans of the body part from different angles.

Where the body part has been scanned using photogrammetry, the method may comprise using the known position of the detectors during the scan to create a 3D model of the body part using the images captured by the detectors.

Where the body part has been scanned using stereo vision, the method may comprise using the known position and separation of the detectors during the scan when comparing the images from the two detectors to determine depth information of the scanned portion of the body part.

Where the body part has been scanned using laser line scanning, the method may comprise analysing the images captured of the body part whilst a laser line is projected onto the body part, and determining topography of the body part from the image of the laser line.

The method may comprise determining the orientation and/or position, for example the 3D orientation and/or position, of the markers on the socket shape. The method may comprise determining the orientation and/or position, for example the 3D orientation and/or position, of the socket shape. The method may comprise determining the orientation and/or position, for example the 3D orientation and/or position, of the socket shape from the 3D position and/or orientation of the markers on the socket shape.

The method may comprise determining the position and/or orientation of the socket shape relative to the scanned body part. The method may comprise using the determined position and/or orientation of the scanned body part to determine the position and/or alignment of the prosthetic components relative to the prosthetic socket. For example, the determined position and orientation of a thigh may be used to align a prosthetic shin and foot with a prosthetic socket, such that when a user places their scanned thigh in the prosthetic socket, the prosthetic shin and foot align with the user's thigh.

The method may comprise determining the shape of the portion of the body part which is in contact with the socket shape from the known shape of the socket shape and the determined position and/or orientation of the socket shape. The method may comprise adding the determined shape of the portion of the body part which is in contact with the socket shape during the scan to the 3D model. This may advantageously allow the model to include portions of the body part which cannot be scanned directly.

The method may comprise matching the shape, size, and/or colour of the prosthetic to the shape, size, and/or colour of a secondary body part, such as a scanned secondary body part. For example, if the prosthetic is a prosthetic leg, the method may comprise matching the prosthetic to the other, intact, leg.

According to a third aspect of the present invention there is provided a computer file, the computer file comprising a scan of a body part, the scan having been made with the apparatus of the first aspect of the present disclosure, and/or with the method of the second aspect of the present disclosure.

The scan of the body part may comprise a 3D digital model of the body part.

According to an fourth aspect of the present disclosure, there is provided a method of designing a prosthesis, the method comprising designing the socket of the prosthesis from a scan of a body part, the scan obtained using the apparatus of the first aspect of the present disclosure, and/or with the method of the second aspect of the present disclosure.

The method may comprise using the computer file of the third aspect of the present disclosure.

Also disclosed herein is a method of manufacturing a prosthesis, the method comprising manufacturing a prosthetic limb designed using the method of the second aspect of the present disclosure.

The method may comprise milling shape captured for prosthesis production.

The method may comprise 3D printing the prosthesis.

Also disclosed herein is a prosthesis, the prosthesis manufactured by the previous disclosure.

It should be understood that the individual features and/or combinations of features defined above in accordance with any aspect of the present invention or below in relation to any specific embodiment of the invention may be utilised, either separately and individually, alone or in combination with any other defined feature, in any other aspect or embodiment of the invention. For example features described in respect of an apparatus may equally apply to a corresponding method, and vice versa.

Furthermore, the present invention is intended to cover apparatus configured to perform any feature described herein in relation to a method and/or a method of using, producing, or manufacturing any apparatus feature described herein.

It will be appreciated that features which are described in relation to singular features may apply equally to some or all of a plurality of that feature. For example, features which are described in relation to a detector may apply to some or all detectors in embodiments comprising more than one detector. It will also be understood that features which are described in relation to a container may be equally applicable to a collapsible container.

### Brief description of the drawings

Figure 1 shows a scanning apparatus for use with pressurised liquid according to the invention;
Figure 2 shows the scanning apparatus in use;
Figure 3 shows part of the scanning apparatus for use with pressurised liquid;
Figure 4 shows a scanning apparatus for use with a flow of fluid;
Figure 5 shows a collapsible scanning apparatus;
Figure 6 shows part of an embodiment of a scanning apparatus according to the present invention;
Figure 7 shows part of another embodiment of a scanning apparatus according to the present invention;
Figure 8 shows a plurality of cameras of a scanning apparatus according to the present invention;
Figure 9 shows a plurality of laser line scanners of a scanning apparatus according to the present invention;
Figure 10 shows another embodiment of a scanning apparatus according to the present invention;

### Detailed description of the drawings

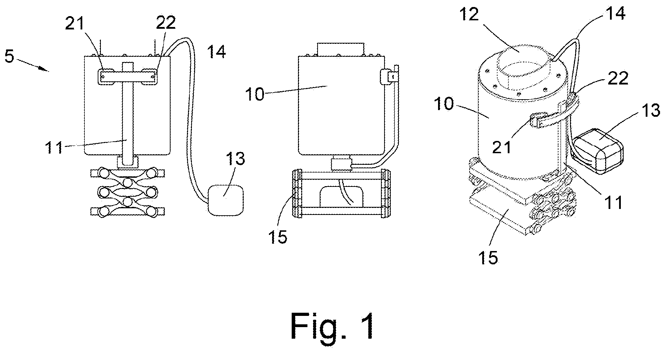

**Figure 1** shows different views of a scanning apparatus 5 (not within the literal scope of claim 1). The scanning apparatus 5 comprises a laser projector 21 and a camera 22 which are both mounted on a rotating frame 11. The scanning apparatus 5 comprises a container 10 and a pumping system 13 to pressurize the contents of the container 10. The number of laser projectors 21 and cameras 22 may be increased to reduce the time required to perform a scan, or may be reduced to reduce the cost of the scanning apparatus 5. The scanning apparatus 5 includes an adjustable mechanism 15. The adjustable mechanism 15 can be used to raise or lower the scanning apparatus 5 for use by different users which helps the user to stay stationary and level while their body part is correctly positioned in the container 10. The container 10 comprises an opening 12. The opening 12 can be adjusted around the body part of a user, to seal the container 10 when a user's body part is inserted into the container 10, and may comprise, e.g., a latex barrier or an inflatable bladder. The fluid (liquid or gas) in the container 10 can then be pressurised with the pump 13 via the pipe 14 to pressurise the soft tissue of the body part. This simulates the loaded condition of the body part and minimises further modification of the captured shape.

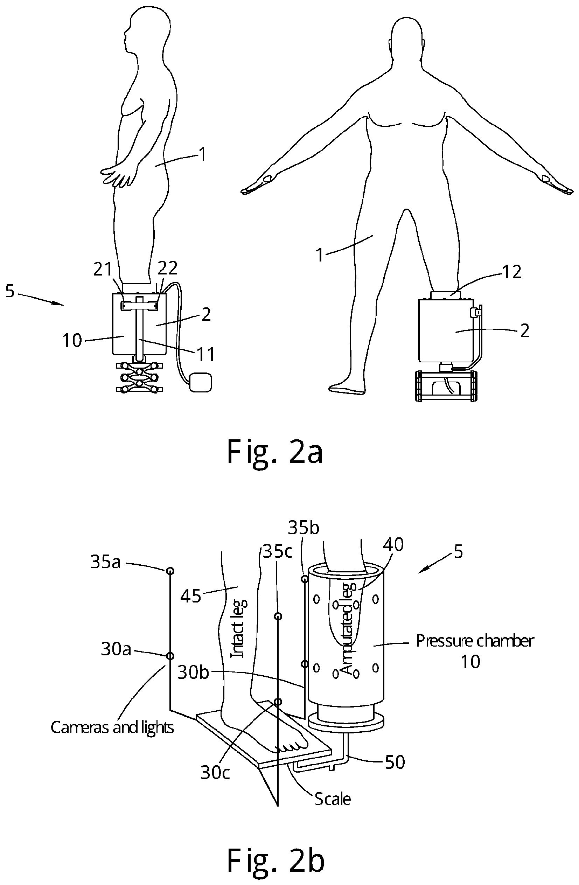

**Figure 2a** shows the scanning apparatus 5 of Figure 1 being used by a user 1 (not within the literal scope of claim 1). The user's 1 residual leg is placed in the container 10. The opening 12 is sealed around the user's 1 residual leg. To perform a scan, the laser projector 21 projects a single vertical line through the container 10 on to the residual leg, and the camera 22 captures an image of the projected line on the surface of the leg. Alternatively, the laser projector 21 may project multiple parallel lines or even a pattern if one uses structured light or photogrammetry. In an alternative embodiment, UV light may be used, for example when using a stereo vision system.

The frame 11 rotates around the container 10, and the camera 22 captures images of the projected line from the laser projector 21 as the frame 11 moves around the container 19. The frame rotates 360° around the container. Images are collected 360° around the residual leg. Image processing software processes each captured image to determine the depth of surface within the line projected from the laser projector 21. By analysing all the images, it is possible to create a 360° computer model of the residual leg. It will be appreciated by one skilled in the art that the speed of the scan can be increased by increasing the number of laser projectors 21 and cameras 22 around the container 10.

**Figure 2b** shows a scanning apparatus 5 being used by a user (not within the literal scope of claim 1). As before, the residual leg 40 is placed in the container 10 and scanned. This scanning apparatus 5 additionally includes secondary cameras 30a-c and secondary light sources 35a-c which scan the intact leg 45. The secondary light sources 35a-c projects light onto the intact leg 45. The secondary cameras 30a-c scan the intact leg 45 while the light from the secondary light sources 35a-c is projected onto the intact leg 45. The intact leg 45 may be scanned using any of the methods of scanning the residual leg 40. The scan of the intact leg 45 can be used to match the design of the prosthetic for the residual leg 40 to the appearance (shape, size, colour, etc) of the intact leg 45.

The scanning apparatus 5 comprises scales 50. By monitoring both pressure and body weight (using scales 50), body weight and pressure can be correlated and/or converted one to the other.

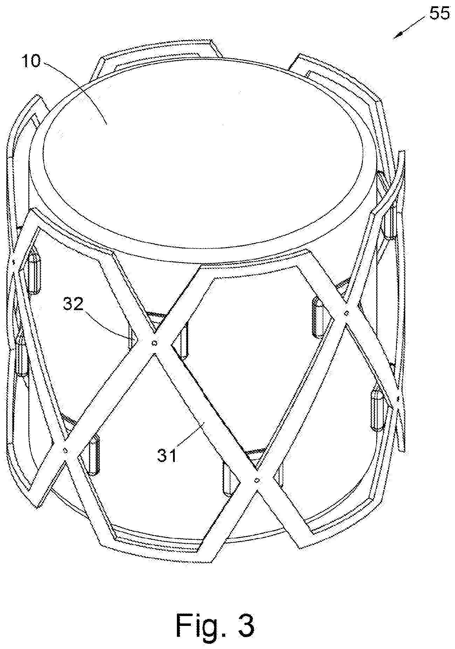

**Figure 3** shows a scanning apparatus 55 comprising a fixed frame 31 and number of cameras 32 around a container 10 (not within the literal scope of claim 1). A user's body part is placed in the container 10 for scanning. The container 10 is sealed, and the fluid (liquid or gas) within is pressurised. When using photogrammetry, the cameras 32 are triggered simultaneously to scan the body part. Because all cameras 32 are triggered simultaneously, a snap shot of the body part at a particular moment in time is captured. This increases the accuracy of the resulting model, as the body part cannot move between the capture of different images.

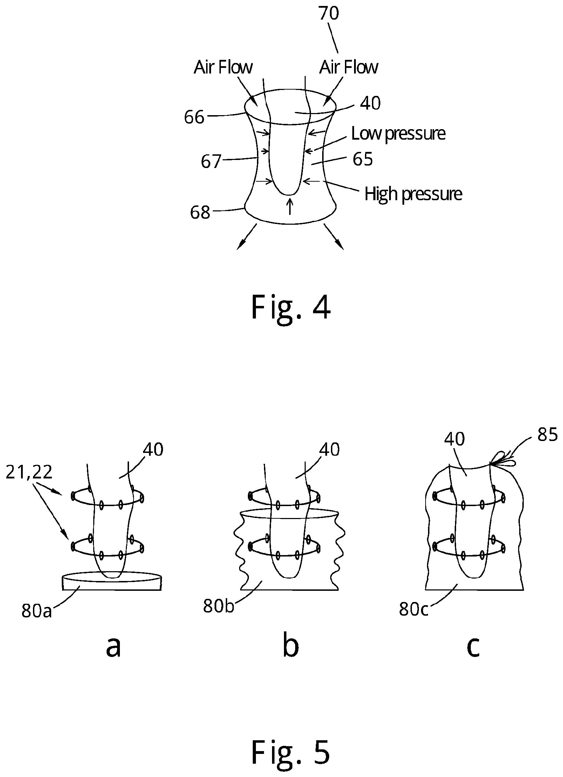

**Figure 4** shows a container 65 for use in a scanning apparatus. The walls of the container 65 are concave, such that the diameter of the container 65 decreases from the top 66 of the container 65 to the middle 67 of the container 65, and the diameter of the container 65 increases from the middle 67 of the container 65 to the bottom 68 of the container 65. In use, a residual limb 40 is placed in the container 65. An air flow 70 is passed through the container 65 from the top 66 of the container 65 to the bottom 68 of the container. Due to the change in the diameter of the container 65 along the direction of air flow 70, the air pressure inside the container 65 changes along the direction of air flow 70. Without wishing to be bound by theory, it is believed that the container 65 may act as a venturi and/or may provide a venturi effect. The top 66 and bottom 68 of the container 65 experience a higher air pressure, and the middle 67 of the container 65 experiences a lower pressure. By controlling the shape of the container 65 it is therefore possible to control the air pressure along the direction of air flow 70 within the container 65. This means that the pressurisation of the soft tissue of the residual leg 40 can be controlled. This in turn means that the loaded conditions which would be experienced by the residual leg 40 when using a prosthetic can be more accurately reproduced within the container 65, and so the scan of the residual leg 40 can be used to produce a more accurate and comfortable prosthetic.

**Figures 5a****-c** show a collapsible container 80 for use in a scanning apparatus. The scanning apparatus comprises two rings of alternating light sources 21 and cameras 22. These rings are stacked one above the other. The rings and/or the light sources 21 and cameras 22 are supported by a frame (not shown). The residual leg 40 is placed within the rings, above the collapsed container 80a. The collapsible container 80b is then extended/deployed over the residual leg 40 and the rings of light sources 21 and cameras 22. Once the collapsible container 80c is fully extended over the residual leg 40, the top of the collapsible container 80c is sealed, which in this embodiment is carried out by use of a draw string 85. It will be appreciated that, in other embodiments not depicted here merely for conciseness, the seal may be comprise or may consist of a latex barrier or an inflatable bladder. The first volume of the collapsible container 80c can then be pressurised. The first volume is pressurised by the body weight of the user through the residual leg 40.

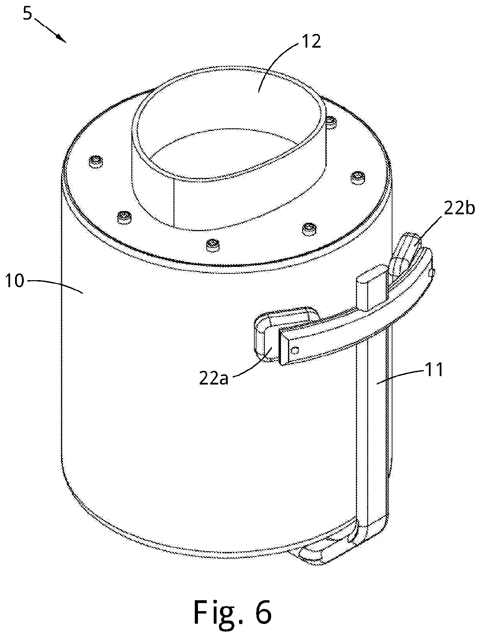

**Figure 6** shows a scanning apparatus 5 configured to scan a user's body part using stereo vision (not within the literal scope of claim 1). The scanning apparatus 5 comprises a container 10. The container 10 comprises an opening 12. The opening 12 can be adjusted around the body part of a user, to seal the container 10 when a user's body part is inserted into the container 10. The scanning apparatus 5 comprises a first camera 22a and a second camera 22b, which are both mounted on a rotating frame 11. The container 10 is transparent, such that the cameras 22a-b can image the contents of the container 10, for example a user's body part which is inserted into the container 10.

The cameras 22a-b are both directed towards the longitudinal axis of the container 10. The two cameras 22a-b are horizontally displaced from each other. To perform stereo vision scanning, the two cameras 22a-b each simultaneously capture an image, for example each camera 22a-b captures an image of a user's body part inserted into the container 10. The fields of view of the cameras 22a-b overlap. Consequently, the two cameras 22a-b capture overlapping images of the body part from different angles. Due to the different angles at which the images of the body part are captured, stereo vision analysis can be performed on the two images of the body part, to determine depth information, similar to binocular depth perception in human vision. From the determined depth perception, it is possible to create a 3D model of the body part using stereo vision from the captured images of the body part.

The frame 11 may rotate around the container 10. The cameras 22a-b may therefore scan other portions of the body part, of the cameras 22a-b may scan completely around the body part, which would allow a full 3D model of the body part to be created.

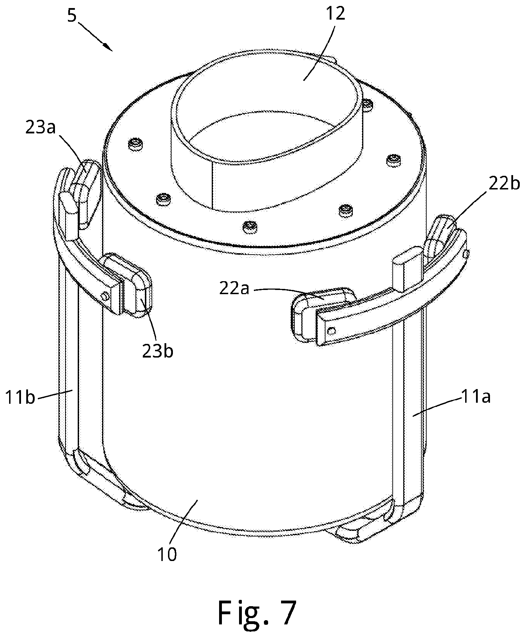

**Figure 7** shows a scanning apparatus 5 configured to scan a user's body part using stereo vision, very similar to the scanning apparatus of Figure 6 (not within the literal scope of claim 1). The scanning apparatus 5 comprises a container 10 with an opening 12. A first pair of cameras 22a-b are mounted on a first rotating frame 11a. Additionally, a second pair of cameras 23a-b are mounted on a second rotating frame 11b. The first pair of cameras 22a-b and the second pair of cameras 23a-b are both configured to capture images for stereo vision scanning. The use of two pairs of cameras allows for faster scanning. The use of two pairs of cameras also allows a larger area of the body part to be scanned simultaneously. Where only one pair of cameras is used, the images of different portions of the body part cannot be captured simultaneously, and so the body part may move between image captures. The use of two pairs of cameras, which can capture images simultaneously, can therefore create a higher quality 3D model, as the body part cannot move between image captures.

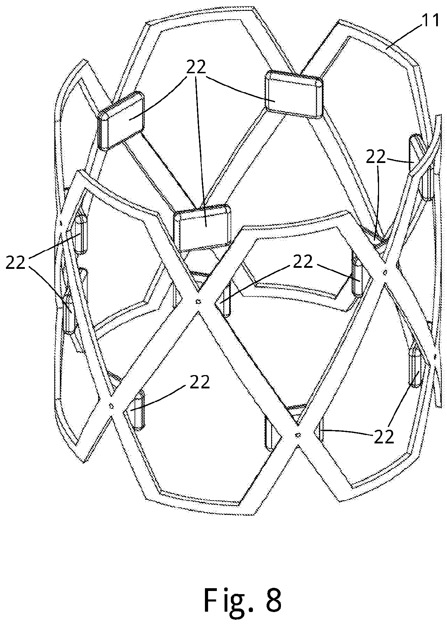

**Figure 8** shows a plurality of cameras 22 configured to scan a user's body part using photogrammetry. There are twelve cameras 22 mounted on a frame 11. The rest of the scanning apparatus, for example the container, are omitted from Figure 8 for clarity, such that the cameras 22 and the frame 11 can clearly be seen. The frame 11 and the cameras 22 may be inside or outside the container.

The cameras 22 are mounted on the frame 11 in a lower ring of six cameras 22 and an upper ring of six cameras 22. The cameras 22 are all directed towards the longitudinal axis of the frame 11. The cameras 22 are arranged such the cameras 22 can simultaneously capture images from all sides of a user's body part that is placed along the longitudinal axis of the frame 11. As the body part is imaged from all sides, the captured images can be combined using photogrammetry techniques to create a complete 360° 3D model of the body part.

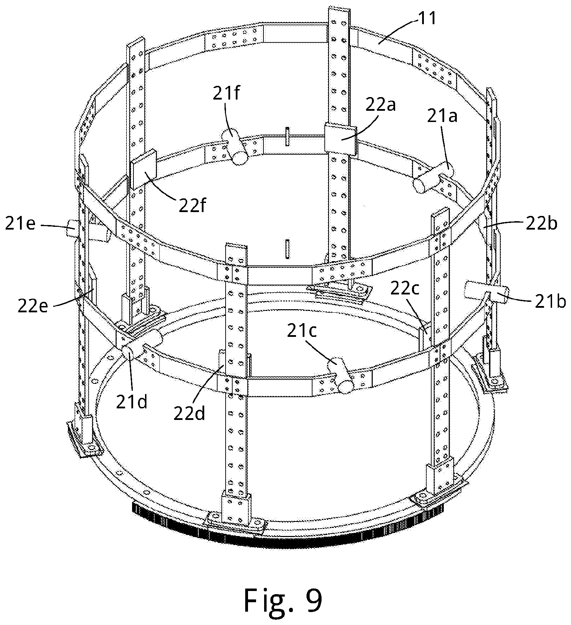

**Figure 9** shows a plurality of laser line scanners configured to scan a user's body part. Each laser scanner comprises a laser projector 21a-f and a camera 22a-f. The laser projectors 21a-f and the cameras 22a-f are mounted on a frame 11. As in Figure 8, the rest of the scanning apparatus, for example the container, are omitted from Figure 9 for clarity, such that the laser projectors 21a-f, the cameras 22a-f and the frame 11 can clearly be seen. The frame 11 and the laser line scanners may be inside or outside the container. The laser projectors 21a-f and the cameras 22a-f are directed towards the longitudinal axis of the frame.

To perform a scan, each laser projector 21a-f projects a vertical line on to a user's body part, and each corresponding camera 22a-f captures an image of the projected line on the surface of the body part. The captures images of the laser lines are analysed to determine the topography of the body part. The frame 11 is rotated around the body part. By rotating the frame 11, the laser projectors 21a-f can project a laser line onto different parts of the body part, and corresponding images can be captured. By rotating the frame like this, it is possible to laser line scan completely around the body part, from which a 3D model of the body part can be created.

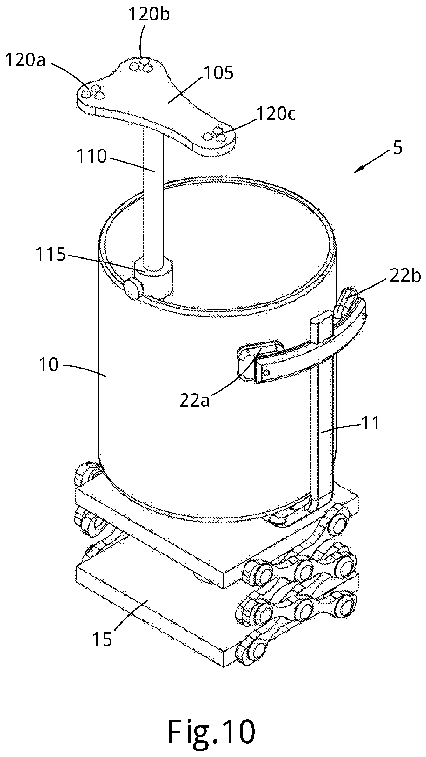

It is difficult to perform transfemoral scans, because it is difficult to scan the proximal structure in the pelvis and femur, such as the ischial tuberosity and the greater trochanter. For example, it is difficult to arrange a laser projector and camera between a user's thighs. One possible solution to this problem is to use socket shapes, as shown in Figure 10.

**Figure 10** shows a scanning apparatus 5 configured to scan a user's body part using stereo vision (not within the literal scope of claim 1). The scanning apparatus 5 comprises a container 10. The scanning apparatus 5 comprises a first camera 22a and a second camera 22b, which are both mounted on a rotating frame 11. The container 10 is transparent, such that the cameras 22a-b can image the contents of the container 10, for example a user's body part which is inserted into the container 10. The container 10 and frame 11 are mounted on an adjustable mechanism 15. The adjustable mechanism 15 can be used to raise or lower the scanning apparatus 5 for use by different users which helps the user to stay stationary and level while their body part is correctly positioned in the container 10.

The scanning apparatus 10 includes a socket shape 105. The socket shape 105 is mounted on a support 110, and the support 110 is attached to the container 10 with an adjustable attachment 115. The socket shape 105 can be raised or lowered be raising or lowering the support 110 through the adjustable attachment 115. The socket shape 105 includes markers 120a-c.

In use, a user would place their residual leg (not shown) in the container. A socket shape corresponding to the shape of the area of the thigh which could not be scanned would be chosen. For example, the socket shape could have a shape corresponding to the shape of the ischial tuberosity. The socket shape would be extended into contact with the area of the user which could not be scanned. The shape of the socket shape would be such that the socket shape would cup the area of the user which could not be scanned.

During the scan, the markers 120a-c on the socket shape 105 would be identified. The position of the markers 120a-c in space relative to the residual leg would then be used to determine the 3D position and orientation of the socket shape in space relative to the residual leg. As the socket shape 105 cups the area of the thigh which cannot be scanned, and the shape, position and orientation of the socket shape 105 are known, it is possible to know the shape, position and orientation of the area of the thigh which cannot be scanned. The shape, position and orientation of the area of the thigh which cannot be scanned can then be included in the 3D model of the residual leg. The 3D model of the residual leg is generated from the scanned parts of the residual leg and from the determined shape, position and orientation of the area of the residual leg which cannot be scanned.

## Claims

1. A scanning apparatus for scanning a body part (40), the scanning apparatus comprising:
a collapsible container (80) configurable to receive the body part (40) and to receive a first fluid, wherein the collapsible container (80) is at least partially flexible; and
a pump,
**characterised in that** the pump is configured to pressurise the first fluid and to apply pressure to or on at least a portion of the body part (40) located in the container (80), and
wherein the collapsible container comprises a sealing means (85), wherein in use the sealing means (85) at least partially seals the collapsible container (80) around or against the body part (40).

2. The scanning apparatus of claim 1, wherein the collapsible container (80) is portable.

3. The scanning apparatus of any preceding claim, wherein the collapsible container (80) is configurable between at least a collapsed configuration (80a) and an extended/deployed configuration (80c).

4. The scanning apparatus of any preceding claim, the scanning apparatus comprising a frame, wherein the frame is deployable between a first or stowed configuration and a second or deployed configuration.

5. The scanning apparatus of claim 4, the scanning apparatus comprising a plate, wherein the frame is mounted or attached to a first side of the plate, and the collapsible container is mounted or attached to the first side of the plate.

6. The scanning apparatus of any preceding claim, wherein the collapsible container (80) is at least partially inflatable and/or the collapsible container (80) is concertinaed.

7. The scanning apparatus of any preceding claim, wherein the first fluid comprises a liquid or a gas.

8. The scanning apparatus of any preceding claim, wherein the collapsible container (80) comprises a bag.

9. The scanning apparatus of any preceding claim, wherein the scanning apparatus is configured to scan the body part (40) to determine shape and/or topography of the body part.

10. A method of scanning a body part (40), the method comprising:
receiving the body part and a first fluid in a collapsible container (80), wherein the collapsible container is at least partially flexible,
sealing the collapsible container at least partially around or against the body part (40),
pressurising the first fluid around the body part using a pump, and
scanning the body part.

11. A method of scanning a body part according to claim 10, further comprising creating a computer file, the computer file comprising a scan of said body part.

12. A method of designing a prosthesis, the method comprising designing the socket of the prosthesis from a scan of a body part, the scan obtained using the method of claim 10.

## Patentansprüche

1. Abtastgerät zum Abtasten eines Körperteils (40), wobei das Abtastgerät Folgendes umfasst:
einen zusammenklappbaren Behälter (80), der konfigurierbar ist, um den Körperteil (40) aufzunehmen und ein erstes Fluid aufzunehmen, wobei der zusammenklappbare Behälter (80) mindestens teilweise flexibel ist; und
eine Pumpe,
**dadurch gekennzeichnet, dass** die Pumpe konfiguriert ist, um das erste Fluid unter Druck zu setzen und Druck auf mindestens einen Teil des in dem Behälter (80) befindlichen Körperteils (40) auszuüben, und
wobei der zusammenklappbare Behälter ein Dichtungsmittel (85) umfasst, wobei das Dichtungsmittel (85) im Gebrauch den zusammenklappbaren Behälter (80) um den Körperteil (40) herum oder gegen diesen mindestens teilweise abdichtet.

2. Abtastgerät nach Anspruch 1, wobei der zusammenklappbare Behälter (80) tragbar ist.

3. Abtastgerät nach einem der vorstehenden Ansprüche, wobei der zusammenklappbare Behälter (80) zwischen mindestens einer zusammengeklappten Konfiguration (80a) und einer ausgefahrenen/entfalteten Konfiguration (80c) konfigurierbar ist.

4. Abtastgerät nach einem der vorhergehenden Ansprüche, wobei das Abtastgerät einen Rahmen umfasst, wobei der Rahmen zwischen einer ersten oder verstauten Konfiguration und einer zweiten oder entfalteten Konfiguration einsetzbar ist.

5. Abtastgerät nach Anspruch 4, wobei das Abtastgerät eine Platte umfasst, wobei der Rahmen an einer ersten Seite der Platte montiert oder befestigt ist und der zusammenklappbare Behälter an der ersten Seite der Platte montiert oder befestigt ist.

6. Abtastgerät nach einem der vorhergehenden Ansprüche, wobei der zusammenklappbare Behälter (80) mindestens teilweise aufblasbar ist und/oder der zusammenklappbare Behälter (80) wie eine Ziehharmonika gefaltet ist.

7. Abtastgerät nach einem der vorhergehenden Ansprüche, wobei das erste Fluid eine Flüssigkeit oder ein Gas umfasst.

8. Abtastgerät nach einem der vorhergehenden Ansprüche, wobei der zusammenklappbare Behälter (80) einen Beutel umfasst.

9. Abtastgerät nach einem der vorhergehenden Ansprüche, wobei das Abtastgerät konfiguriert ist, um den Körperteil (40) abzutasten, um die Form und/oder Topographie des Körperteils zu bestimmen.

10. Verfahren zum Abtasten eines Körperteils (40), wobei das Verfahren Folgendes umfasst:
Aufnehmen des Körperteils und eines ersten Fluids in einem zusammenklappbaren Behälter (80), wobei der zusammenklappbare Behälter mindestens teilweise flexibel ist,
Abdichten des zusammenklappbaren Behälters mindestens teilweise um den Körperteil (40) herum oder gegen diesen,
Beaufschlagen des ersten Fluids um den Körperteil herum mit Druck unter Verwendung einer Pumpe, und
Abtasten des Körperteils.

11. Verfahren zum Abtasten eines Körperteils nach Anspruch 10, ferner umfassend das Erstellen einer Computerdatei, wobei die Computerdatei eine Abtastung des Körperteils umfasst.

12. Verfahren zum Konstruieren einer Prothese, wobei das Verfahren das Konstruieren des Sockels der Prothese anhand einer Abtastung eines Körperteils umfasst, wobei die Abtastung unter Verwendung des Verfahrens nach Anspruch 10 erhalten wird.

## Revendications

1. Appareil de scannage pour scanner une partie de corps (40), l'appareil de scannage comprenant :
un contenant pliable (80) pouvant être configuré pour recevoir la partie de corps (40) et pour recevoir un premier fluide, dans lequel le contenant pliable (80) est au moins partiellement souple ; et
une pompe,
**caractérisé en ce que** la pompe est configurée pour mettre sous pression le premier fluide et pour appliquer une pression sur au moins une partie de la partie de corps (40) qui est localisée dans le contenant (80), et
dans lequel le contenant pliable comprend un moyen d'étanchéité (85), dans lequel, en cours d'utilisation, le moyen d'étanchéité (85) assure l'étanchéité au moins partielle du contenant pliable (80) autour de la partie de corps (40) ou contre celle-ci.

2. Appareil de scannage selon la revendication 1, dans lequel le contenant pliable (80) est portatif.

3. Appareil de scannage selon l'une quelconque des revendications précédentes, dans lequel le contenant pliable (80) peut être configuré entre au moins une configuration pliée (80a) et une configuration étendue/déployée (80c).

4. Appareil de scannage selon l'une quelconque des revendications précédentes, l'appareil de scannage comprenant une structure, dans lequel la structure peut être déployée entre une première configuration ou configuration repliée et une seconde configuration ou configuration déployée.

5. Appareil de scannage selon la revendication 4, l'appareil de scannage comprenant une plaque, dans lequel la structure est montée sur un premier côté de la plaque ou lui est liée, et le contenant pliable est monté sur le premier côté de la plaque ou lui est lié.

6. Appareil de scannage selon l'une quelconque des revendications précédentes, dans lequel le contenant pliable (80) est au moins partiellement gonflable et/ou le contenant pliable (80) est plié en accordéon.

7. Appareil de scannage selon l'une quelconque des revendications précédentes, dans lequel le premier fluide comprend un liquide ou un gaz.

8. Appareil de scannage selon l'une quelconque des revendications précédentes, dans lequel le contenant pliable (80) comprend une poche.

9. Appareil de scannage selon l'une quelconque des revendications précédentes, dans lequel l'appareil de scannage est configuré pour scanner la partie de corps (40) afin de déterminer la forme et/ou la topographie de la partie de corps.

10. Procédé de scannage d'une partie de corps (40), le procédé comprenant :
la réception de la partie de corps et d'un premier fluide dans un contenant pliable (80), dans lequel le contenant pliable est au moins partiellement souple ;
la réalisation de l'étanchéité du contenant pliable au moins partiellement autour de la partie de corps (40) ou contre celle-ci ;
la mise sous pression du premier fluide autour de la partie de corps en utilisant une pompe ; et
le scannage de la partie de corps.

11. Procédé de scannage d'une partie de corps selon la revendication 10, comprenant en outre la création d'un fichier informatique, le fichier informatique comprenant un scan de ladite partie de corps.

12. Procédé de conception d'une prothèse, le procédé comprenant la conception de l'emboîture de la prothèse à partir d'un scan d'une partie de corps, le scan étant obtenu en utilisant le procédé selon la revendication 10.
